# EUROPEAN PATENT SPECIFICATION

(11) **EP 4 292 557 B1**
(45) Date of publication and mention of the grant of the patent: **23.07.2025**
(21) Application number: 23179902.4
(22) Date of filing: 16.06.2023
(51) Int. Cl.: A61B 34/10, A61C 7/00

(54) **METHOD, SERVER, AND COMPUTER PROGRAM FOR PROVIDING SURGERY SIMULATION**
VERFAHREN, SERVER UND COMPUTERPROGRAMM ZUR BEREITSTELLUNG EINER CHIRURGISCHEN SIMULATION
PROCÉDÉ, SERVEUR ET PROGRAMME INFORMATIQUE POUR FOURNIR UNE SIMULATION CHIRURGICALE

(30) Priority: 17.06.2022 KR 20220073926
(43) Date of publication of application: 20.12.2023
(73) Proprietor: 3D ONS, INC., Seoul 06023 (KR)
(72) Inventor: CHOE, Won Seok, 06023 Seoul (KR)
(74) Representative: Walaski, Jan Filip

(56) References cited:
- KR-B1- 102 226 467
- US-A1- 2020 197 137
- US-A1- 2021 315 667

## Description

### CROSS-REFERENCE TO RELATED APPLICATION

This application claims priority to Korean Patent Application No. 2022-0073926, filed on JUN 17, 2022.

### BACKGROUND

### 1. Field of the Invention

Various embodiments of the present disclosure relate to surgery simulations, and more particularly, to a method, a server, and a computer program for providing a surgery simulation.

### 2. Discussion of Related Art

Malocclusion refers to the misalignment of teeth for a certain reason or an occlusal irregularity causing an aesthetic or functional problem. Malocclusion may cause functional problems such as problems in masticatory movements and pronunciation, and cause not only aesthetic problems in one's appearance but also health problems such as cavities and gum diseases.

When a malocclusion is corrected using only teeth, aesthetic and functional problems can be fixed only when a patient's facial skeleton does not significantly deviate from an ideal position. When there is a problem with the patient's facial skeleton, the problem cannot be fixed by simply correcting the arrangement of the teeth and thus the position of the facial skeleton should be improved through orthognathic surgery. Orthognathic surgery is performed to fix an anatomical problem of the patient (e.g., asymmetric lower jaw, overdeveloped lantern jaw, or deformed small jaw that has not grown due to trauma) by correcting the position, shape or the like of the jaw.

In the case of orthognathic surgery, an osteotomy (the surgical cutting of bone to reset an angle and position thereof) is performed and thus it is necessary to determine whether a patient needs to get the orthognathic surgery through a detailed diagnosis, sufficiently explain the determination to the patient, and plan a surgery accurately to reflect the patient's decision. To this end, it will be very helpful to provide the patient with a medical consultation to explain the surgery plan through a virtual simulation of a surgery plan, so that the patient may easily understand his or her problem. Through a virtual surgery simulation, a doctor can provide the patient with various types of clear information to help the patient's decision-making. Korean Laid-open Patent Application No. 10-2016-0043411 discloses a method of performing a virtual orthodontic surgery simulation.

However, in a surgery simulation of the related art, an arbitrary origin of rotation (e.g., the center of the volume of the jawbone, a point on a screen designated by a user with a mouse during a simulation, etc.) and an x-axis, a y-axis, and a z-axis, which are ambiguous criteria (e.g., preset fixed axes on a simulation screen that represent a left and right direction, a front and back direction, an up and down direction, etc.), are used, and thus, anatomical features of each patient are not reflected and clear criteria are not provided.

In such an environment, the movement and rotation of three-dimensional (3D) spatial data displayed on a two-dimensional (2D) monitor would not be an easy task for even professionals specialized in the task. Therefore, during the planning of a surgery, it will be difficult and require a lot of effort for a doctor to simulate movement and rotation on the x, y, and z axes by him or herself so as to set an ideal position of the jawbone for orthognathic surgery.

In addition, in existing systems, all movements and rotations performed by a user (e.g., a person who will perform a surgery) are merged into a movement result and a rotation result and thus there are only two stages, i.e., the stage before orthognathic surgery and the stage after the orthognathic surgery. Thus, a clear explanation cannot be provided to a patient and the user cannot perform the same plan again.

Accordingly, in the art to which the present disclosure pertains, there is a need for research and development of a simulation to which diagnosis factors, such as an origin of rotation and axes of rotation, customized for a patient according to an anatomical shape of the patient, are applied, a simulation configured systematically to explain the goal and progress of a surgery to a patient, and a simulation for achieving the same result each time using various numerical values.

US 2020/197137 A1 discloses systems and methods for computer-aided orthognathic surgical planning. KR 102226467 B1 discloses a simulation apparatus and a method for orthognathic surgery, in which a jaw correction surgery is simulated by analyzing the skull image of a patient who needs jaw correction to divide the maxillary, mandible, and mesial bone pieces constituting the jaw into individual structures, and perform at least one of movement and rotation for each structure, wherein the simulated surgery comprises a plurality of operations of translation and rotation of the individual structures along predefined X, Y, and Z axes based on an anatomical landmark. KR 102226467 B1 does not disclose the third operation comprising the rotation of the object about a second landmark and the third axis being a line segment generated on the basis of the first landmark and the second landmark, the fourth operation comprising moving the object on the second axis, and the fifth operation comprising rotating the object by setting the first landmark as an origin of rotation and the first axis as an axis of rotation.

US 2021/315667 A1 discloses systems and methods for determining orthodontic treatment.

### SUMMARY OF THE INVENTION

To address the aforementioned background art, the present disclosure is directed to providing an orthognathic simulation designed to systematically plan operations of a surgery on the basis of singularities (landmarks, e.g., the anterior nasal spine (ANS)) of an anatomical structure of each patient, so that the goal of a surgery and the like may be explained between a doctor and the patient or between doctors and the same result may be reproduced through numerical values for each of the operations.

A method of providing a surgery simulation according to claim 1 is provided. Optional features are set out in the dependent claims. Moreover, an apparatus according to claim 7 is provided, and a computer program according to claim 8 is provided.

Other aspects of the present disclosure will be apparent from the detailed description and drawings.

### BRIEF DESCRIPTION OF THE DRAWINGS

The above and other objects, features and advantages of the present disclosure will become more apparent to those of ordinary skill in the art by describing in detail exemplary embodiments thereof with reference to the accompanying drawings, in which:
FIG. 1 is a diagram schematically illustrating a system for performing a method of providing a surgery simulation according to an embodiment of the present disclosure;
FIG. 2 illustrates a hardware configuration of a server for performing a method of providing a surgery simulation according to an embodiment of the present disclosure;
FIG. 3 is a flowchart of a method of providing a surgery simulation according to an embodiment of the present disclosure;
FIG. 4 is a diagram illustrating three-dimensional (3D) modeling data according to an embodiment of the present disclosure;
FIG. 5 is a diagram illustrating first to sixth operations of a surgery according to an embodiment of the present disclosure;
FIG. 6 is a diagram illustrating a simulation of each of the first to sixth operations according to an embodiment of the present disclosure;
FIG. 7 is a diagram illustrating a user interface according to an embodiment of the present disclosure;
FIG. 8 is a diagram illustrating a diagnosis index display screen according to an embodiment of the present disclosure; and
FIG. 9 is a diagram illustrating a process of generating splint modeling data according to an embodiment of the present disclosure.

### DETAILED DESCRIPTION OF EXEMPLARY EMBODIMENTS

Hereinafter, various embodiments will be described with reference to the accompanying drawings. In the present specification, various examples are presented to provide an understanding of the present disclosure. However, it will be clear that these embodiments can be implemented without such a detailed description.

The term "component," "module," "system" or the like, when used herein, refers to computer-related entities, hardware, firmware, software, a combination of software and hardware, or execution of software. For example, a component may be understood as, but is not limited to, a procedure performed by a processor, a processor, an object, an execution thread, a program, and/or a computer. For example, both an application executed by a computing device and the computing devices may be components. One or more components may reside in a processor and/or an execution thread. One component may be localized in one computer. One component may be distributed between two or more computers. These components may be run from various computer-readable media storing various data structures therein. Components may communicate with each other through local and/or remote processing, for example, according to a signal containing one or more data packets (e.g., data transmitted from a system through a network such as the Internet according to data and/or a signal from one component interacting with another component in a local or distributed system).

In addition, the term "or" is intended to mean an inclusive "or" rather than an exclusive "or." That is, "X uses A or B" is intended to mean one of the natural implicit substitutions unless otherwise specified or contextually clear. That is, "X uses A or B" should be understood to mean that X uses A, X uses B, or X uses both A and B. The term "and/or" when used herein should be understood to refer to and include all possible combinations of one or more of relevant items listed herein.

In addition, "comprise" and/or "comprising" should be understood to mean the presence of a corresponding feature and/or component. However, it will be understood that the terms "comprise" and/or "comprising" do not preclude the presence or addition of one or more other features, components, and/or groups thereof. Each singular form described in the detailed description and claims should be understood to generally mean "one or more" unless otherwise specified or contextually clear as indicating a singular form.

It will be understood by those of ordinary skill in the art that various examples of logical blocks, configurations, modules, circuits, means, logic, and operations of an algorithm additionally described below in relation to embodiments set forth herein can be implemented by electronic hardware, computer software, or a combination thereof. To clearly indicate the interchangeability of hardware and software, various examples of components, blocks, configurations, means, logic, modules, circuits, and operations have generally been described above in terms of functionalities thereof. Whether to implement such functionality by hardware or software depends on specific applications and design limitations imposed on an overall system. It will be obvious that described functionalities for each specific application can be implemented in various ways by skilled technicians. However, it should not be understood that decisions of such implementation are not within the scope of the present disclosure.

A description of embodiments set forth herein is provided to help those of ordinary skill in the art use or implement the present disclosure. It will be apparent to those of ordinary skill in the art that various modifications can be made in these embodiments. General principles defined herein may apply to other embodiments without departing from the scope of the present disclosure. Therefore, the present disclosure is not limited to embodiments set forth herein. The present disclosure should be interpreted within the broadest range consistent with principles and novel features described herein.

As used herein, the term "computer" should be understood to mean various types of hardware devices, including at least one processor, and may be understood to include a software component operating in a corresponding hard device according to an embodiment. For example, a computer may be understood to include, but is not limited to, a smart phone, a tablet PC, a desktop computer, a laptop computer, and a user client and an application running on each device.

Hereinafter, embodiments of the present disclosure will be described in detail with reference to the accompanying drawings.

Operations described herein will be described as being performed by a computer, but a subject of each of the operations is not limited thereto and at least some of the operations may be performed by different devices according to an embodiment.

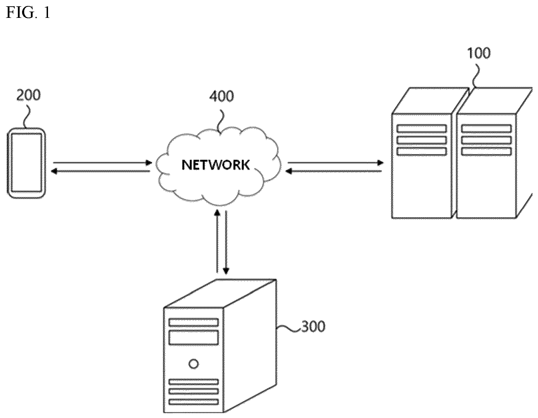

FIG. 1 is a diagram schematically illustrating a system for performing a method of providing a surgery simulation according to an embodiment of the present disclosure.

As illustrated in FIG. 1, a system according to embodiments of the present disclosure may include a server 100, a user terminal 200, an external server 300, and a network 400. The components shown in FIG. 1 are only examples and thus other components may be added or some of the components shown in FIG. 1 may be omitted. The server 100, the external server 300, and the user terminal 200 according to embodiments of the present disclosure may transmit or receive data, for the system according to an embodiment of the present disclosure, to or from one another through the network 400.

The network 400 according to embodiments of the present disclosure may use various types of wired communication systems such as a Public Switched Telephone Network (PSTN), x Digital Subscriber Line (xDSL), Rate Adaptive DSL (RADSL), Multi-rate DSL (MDSL), Very High Speed DSL (VDSL), Universal Asymmetric DSL (UADSL), High Bit Rate DSL (HDSL), and a local area network (LAN).

The network 400 described herein may further use various types of wireless communication systems such as Code Division Multi Access (CDMA), Time Division Multiple Access (TDMA), Frequency Division Multi Access (FDMA), Orthogonal Frequency Division Multi Access (OFDMA), Single Carrier-FDMA (SC-FDMA) and other systems.

The network 400 according to embodiments of the present disclosure may be configured regardless of the type of communication, e.g., wired communication or wireless communication, and configured as various types of communication networks such as a personal area network (PAN) and a wide area network (WAN). In addition, the network 400 may be the well-known World Wide Web (WWW) and may use wireless transmission technology, e.g., Infrared Data Association (IrDA) or Bluetooth, used for short-range communication. Technologies described herein may be used not only in the networks described above but also in other networks.

According to an embodiment of the present disclosure, the server 100 performing a method of providing a surgery simulation (hereinafter referred to as the "server 100") may provide surgery simulation information. For example, the surgery simulation information may be related to virtual surgery related to dental treatment. Specifically, the server 100 may generate three-dimensional (3D) modeling data corresponding to a medical image and provide a user interface including the 3D modeling data. Here, the user interface may be intended to obtain various types of information necessary to perform a surgery simulation from a user of the user terminal 200 or to provide data generated as a result of performing the surgery simulation. The server 100 may perform a virtual surgery simulation related to dental treatment (e.g., double-jaw surgery, jaw-end surgery, etc.) on the basis of a response to the user interface, received from the user terminal 200.

In an embodiment, the server 100 may perform a surgery simulation on the basis of a specific landmark in the 3D modeling data. The landmark represents an anatomical point in the head of a patient, and examples thereof may include, but are not limited to, the anterior nasal spine (ANS) of the maxillary bone, the upper 1 crown point (U1CP) of the front teeth of the upper jaw, the orbitale, the porion, etc.

For example, when a surgery simulation is performed using an origin of rotation and axes that are arbitrarily set by a user or according to a preset system, there may be difficulty in performing ideal surgical correction on the actual anatomical structure of a patient.

As a concrete example, when a user performs a simulation using, as an origin of rotation, an arbitrary point on a screen designated with a mouse or the like on a screen of the simulation or using x, y and z axes (e.g., preset fixed axes representing left and right, front and back, up and down, etc. on the screen of the simulation), it may be difficult to accurately move a specific object to a desired position on the 3D modeling data, thereby preventing the surgery simulation from being smoothly performed.

Accordingly, in the disclosed embodiment, a user can perform rotation and movement with respect to an origin of rotation and axes generated using an anatomical positional relationship of the patient on the basis of a clear criterion (anatomical point). That is, the server 100 may provide a virtual surgery simulation using optimal axes and origin of rotation that are differently set for each patient on the basis of an actual anatomical position and shape of each patient rather than arbitrary axes and an arbitrary origin of rotation.

Accordingly, a user can accurately move a specific object to a desired position on the 3D modeling data to easily perform the surgery simulation, so that the surgery may be easily reproduced and explained.

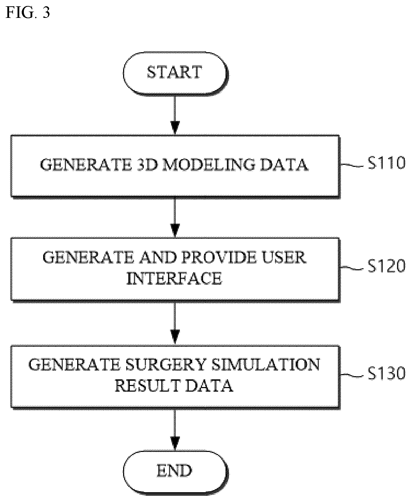

In an embodiment, the server 100 may perform the surgery simulation on the basis of a plurality of operations of a surgery, and generate various types of simulation result data related to the progress of the surgery for each of the plurality of operations. Therefore, a patient-customized surgery simulation can be performed while reflecting current anatomical features of each patient, and rotation and movement can be performed for each of operations of a surgery during the simulation so that the goal of each rotation and movement intended by a person (e.g., a doctor) who will perform a surgery can be checked to systematically plan the surgery. The surgery simulation provided by the server 100 will be described in detail with reference to FIG. 3 below.

Although FIG. 1 illustrates only one server 100 according to an embodiment, it will be apparent to those of ordinary skill in the art that more than one server falls within the scope of the present disclosure and the server 100 may include additional components. That is, the server 100 may include a plurality of computing devices. In other words, a set of nodes may constitute the server 100.

According to an embodiment of the present disclosure, the server 100 may be a server that provides a cloud computing service. More specifically, the server 100 may be a server that provides a cloud computing service for processing information using a computer connected to the Internet other than a user's computer, i.e., a type of Internet-based computing. The cloud computing service may be a service for storing data on the Internet and allowing a user to use desired data or a program regardless of time and place by connecting to the Internet without storing the desired data or program in the user's computer, and the data stored on the Internet can be easily shared and delivered through simple manipulation and clicking. In addition, the cloud computing service may be a service for allowing a desired task to be performed using functions of an application program provided on the web without additionally installing a program and allowing several persons to perform a task at the same time while sharing a document, as well as simply storing data in a server on the Internet. The cloud computing service may be implemented in the form of at least one of Infrastructure as a Service (IaaS), Platform as a Service (PaaS), Software as a Service (SaaS), a virtual machine-based cloud server, or a container-based cloud server. That is, the server 100 of the present disclosure may be implemented in the form of at least one of the above-described cloud computing services. The above-described cloud computing services are only examples and may include a platform for constructing a cloud computing environment of the present disclosure.

The user terminal 200 according to an embodiment of the present disclosure may be understood as a type of node(s) in a system having a mechanism for communication with the server 100. The user terminal 200 is a terminal capable of receiving information about a surgery simulation through the exchange of information with the server 100 and may be understood as a user's terminal. For example, the user terminal 200 may be a terminal related to a user (e.g., a doctor) who desires to obtain surgery guide information through a surgery simulation for orthognathic surgery.

In an embodiment, the user terminal 200 may be connected to the server 100 through the network 400, and provide the server 100 with a plurality of pieces of image data (e.g., 2D image data and 3D image data) and receive a user interface including 3D modeling information or surgery simulation result data in response to the plurality of pieces of image data.

The user terminal 200 may be understood as a type of entity(s) in a system having a mechanism for communication with the server 100. Examples of the user terminal 200 may include a personal computer (PC), a notebook computer, a mobile terminal, a smart phone, a tablet PC, a wearable device, etc., and include various types of devices capable of accessing a wired/wireless network. Examples of the user terminal 200 may include a server implemented by at least one of an agent, an application programming interface (API) or a plug-in. In addition, examples of the user terminal 200 may include an application source and/or a client application.

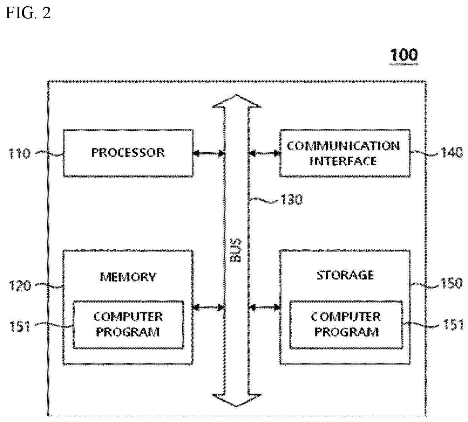

In an embodiment, the external server 300 may be connected to the server 100 through the network 400, and provide various types of information/data necessary to perform the method of providing a surgery simulation or supplied with resulting data generated as the server 100 performs the method of providing a surgery simulation, and store and manage the resulting data. For example, the external server 300 may be a storage server provided separately outside the server 100 but is not limited thereto. Hereinafter, a hardware configuration of the server 100 that performs the method of providing a surgery simulation will be described with reference to FIG. 2.

FIG. 2 illustrates a hardware configuration of a server for performing a method of providing a surgery simulation according to an embodiment of the present disclosure.

Referring to FIG. 2, a server 100 that performs a method of providing a surgery simulation according to an embodiment of the present disclosure may include one or more processors 110, a memory 120 configured to load a computer program 151 to be executed by the one or more processors 110, a bus 130, a communication interface 140, and a storage 150 storing the computer program 151. Here, FIG. 2 illustrates only components related to the embodiment of the present disclosure. Therefore, it will be apparent to those of ordinary skill in the art that other general-purpose components may be further provided, as well as the components illustrated in FIG. 2.

According to an embodiment of the present disclosure, the processor 110 may generally control the overall operation of the server 100. The processor 110 may process signals, data, information, and the like that are input or output through the components described above or may execute an application program stored in the memory 120 to provide appropriate information or functions to a user or a user terminal or process the information or functions.

The processor 110 may perform an operation on at least one application or program for performing methods according to embodiments of the present disclosure, and the server 100 may include one or more processors.

According to an embodiment of the present disclosure, the processor 110 may include one or more cores, and include a processor for analyzing data and performing deep learning, e.g., a central processing unit (CPU), a general-purpose graphics processing unit (GPGPU) or a tensor processing unit (TPU).

The processor 110 may read a computer program stored in the memory 120 and perform a method of providing a surgery simulation according to an embodiment of the present disclosure.

In various embodiments, the processor 110 may further include a random access memory (RAM) (not shown) and a read-only memory (ROM) (not shown) for temporarily and/or permanently storing signals (or data) processed in the processor 110. The processor 110 may be in the form of a system-on-chip (SoC) including at least one of a graphics processor, a RAM, and a ROM.

The memory 120 stores various types of data, instructions, and/or information. The memory 120 may load the computer program 151 from the storage 150 to perform methods/operations according to various embodiments of the present disclosure. When the computer program 151 is loaded in the memory 120, the processor 110 may execute one or more instructions constituting the computer program 151 to perform the methods/operations. The memory 120 may be embodied as a volatile memory such as a RAM but the technical scope of the present disclosure is not limited thereto.

The bus 130 provides a communication function between the components of the server 100. The bus 130 may be embodied as a various types of buses such as an address bus, a data bus, and a control bus.

The communication interface 140 supports wired/wireless Internet communication of the server 100. The communication interface 140 may support various communication methods other than Internet communication. To this end, the communication interface 140 may include a communication module well known in the technical field of the present disclosure. In some embodiments, the communication interface 140 may be omitted.

The storage 150 may store the computer program 151 non-temporarily. When a process of providing a surgery simulation is performed through the server 100, the storage 150 may store various types of information required to provide the process of providing the surgery simulation.

The storage 150 may include a non-volatile memory, such as a ROM, an erasable programmable ROM (EPROM), an electrically erasable programmable ROM (EEPROM) or a flash memory, a hard disk, a detachable disk, or any type of computer-readable recording medium well known in the technical field to which the present disclosure pertains.

The computer program 151 may include one or more instructions causing the processor 110 to perform a method/operations according to various embodiments of the present disclosure when the computer program 151 is loaded in the memory 120. That is, the processor 110 may execute the one or more instructions to perform the method/operations according to various embodiments of the present disclosure.

In an embodiment, the computer program 151 may include one or more instructions for performing a method of providing a surgery simulation including generating 3D modeling data on the basis of a medical image, generating a user interface including the 3D modeling data and providing the user interface to a user terminal, and performing the surgery simulation on the basis of a response to the user interface, which is received from the user terminal, to generate surgery simulation result data.

The operations of the method or an algorithm described above in connection with embodiments of the present disclosure may be implemented directly by hardware, a software module executed by hardware, or a combination thereof. The software module may reside in a RAM, a ROM, an EPROM, an EEPROM, a flash memory, a hard disk, a removable disk, a CD-ROM, or a type of computer-readable recording medium well-known in the technical field to which the present disclosure pertains.

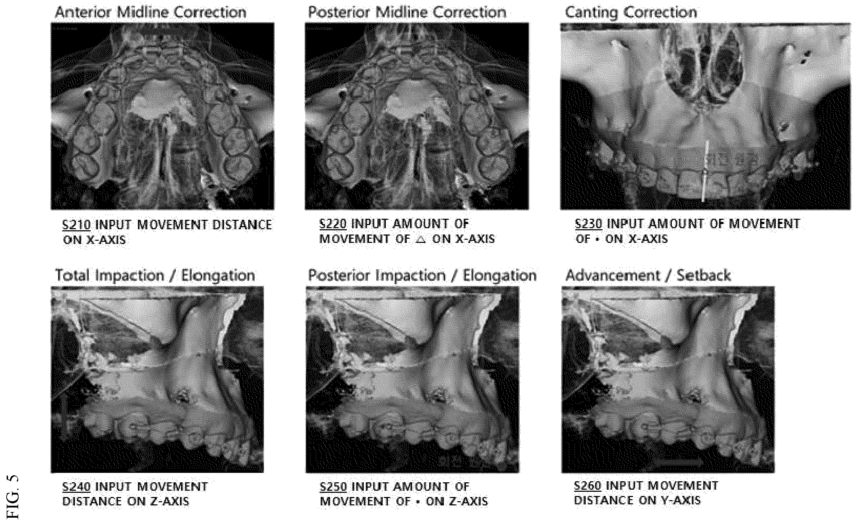

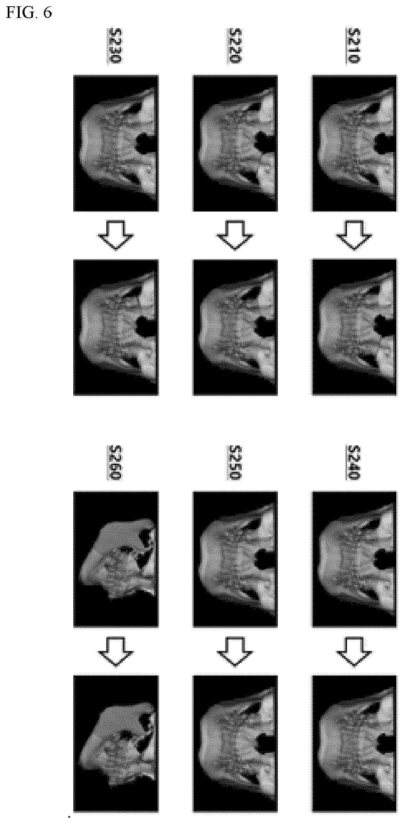

Components of the present disclosure may be embodied in the form of a program (or an application) and stored in a medium to be executed in combination with a computer which is hardware. The components of the present disclosure may be implemented by software programming or software elements, and similarly, embodiments may be implemented in a programming or scripting language such as C, C++, Java, or an assembler, including data structures, processes, routines, or various algorithms which are combinations of other programming components. Functional aspects may be embodied as an algorithm executable by one or more processors. Hereinafter, a method of providing a surgery simulation, which is performed by the server 100, will be described in detail with reference to FIGS. 3 to 9.

FIG. 3 is a flowchart of a method of providing a surgery simulation according to an embodiment of the present disclosure. An order of operations illustrated in FIG. 3 may be changed as necessary and at least one operation may be omitted or added. That is, the operations to be described below are only examples of the present disclosure and the scope of the present disclosure is not limited thereto.

According to an embodiment of the present disclosure, the server 100 may generate 3D modeling data on the basis of a medical image (S110).

In an embodiment, the server 100 may obtain the medical image. In an embodiment, the server 100 may receive the medical image through the communication interface 140. The medical image may be, for example, a CT image obtained by photographing the head of a patient. The receiving of the medical image according to an embodiment of the present disclosure may include receiving or loading a medical image stored in the memory 120. The receiving of the medical image may include receiving raw data from an additional processing module of the same computing device or another computing device or loading raw data into another storage medium on the basis of a wired/wireless communication device.

Specifically, the server 100 may identify one or more landmarks in the medical image. For example, one or more landmarks according to the present disclosure may be identified through an image recognition model or determined based on a user input related to the medical image but are not limited thereto. Here, the image recognition model may be a neural network model trained to identify a specific area (e.g., a landmark) in an image.

In an embodiment, the server 100 of the present disclosure may provide a user interface for setting a landmark (e.g., a landmark recognition user interface) to the user terminal 200, and the user of the user terminal 200 may set a landmark in relation to a specific area of an image using the user interface. The specific description of the method of setting a landmark described above is only an example, and the present disclosure is not limited thereto.

A subject performing embodiments to be described below will be described as the server 100 but is not limited thereto, and at least some or all of operations of each of the embodiments may be performed by another device. For example, embodiments to be described below may also be performed by the user terminal 200.

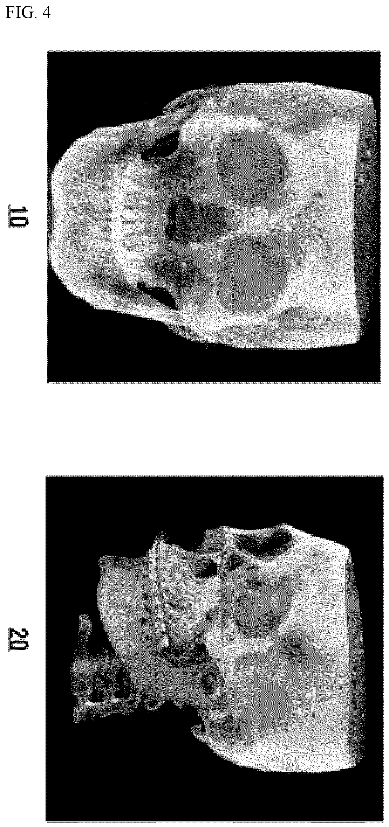

In addition, the server 100 may perform segmentation on medical image data 10 of FIG. 4. In the disclosed embodiment, medical image data may be volume rendering data as shown in FIG. 4 but is not limited thereto. The server 100 may generate 3D modeling data 20 of FIG. 4 on the basis of a segmentation result. In the disclosed embodiment, the 3D modeling data may be mesh data but is not limited thereto. For example, the server 100 may segment upper and lower jaw parts and generate the 3D modeling data 20 of FIG. 4 on the basis of a segmentation result.

In addition, the server 100 may obtain result data to which an osteotomy is applied according to the type of target surgery. For example, a mandibular osteotomy may be needed for orthognathic surgery, and the server 100 may obtain information about an osteomized area of the jawbone according to the type of surgery. The information about the osteomized area may be input directly by a user or be obtained through a selection input for selecting one of the osteotomy menus included in a provided user interface. The server 100 may determine an object, which is to be moved to perform a surgery simulation from the result data to which an osteotomy is applied. For example, the object to be moved may be a specific area of the jawbone to be moved and fixed for orthognathic surgery.

For example, segmentation and an osteotomy related to upper and lower jaw parts may be performed, based on a deep learning model (e.g., a segmentation model) or a user input. The 3D modeling data 20 of FIG. 4 is a target of a virtual surgery simulation and may be 3D data related to the head of an actual patient. Thereafter, the server 100 may perform the surgery simulation by rotating and moving the object to be moved on the segmented and osteomized 3D modeling data 20 of FIG. 4.

According to an embodiment of the present disclosure the server 100 may generate a user interface including 3D modeling data and provide the user interface to the user terminal 200 (S120).

In an embodiment, the user interface 500 may provide a surgery simulation related to a plurality of operations of a surgery by setting axes and reference points corresponding to 3D modeling data on the basis of one or more landmarks. A method of setting axes and reference points corresponding to 3D modeling data will be described in detail below.

That is, the user interface 500 may provide a surgery simulation related to a plurality of operations of a surgery by setting axes and reference points matching a patient on the basis of one or more landmarks.

In an embodiment, the axes and the reference points matching the patient (i.e., axes and reference points corresponding to 3D modeling data of the patient) may include at least one of a point, a line or a plane generated through an operation related to at least one of a midpoint, an intersection, a line segment, a plane, a vertical relationship, or a parallel relationship based on one or more landmarks.

As a concrete example, it is assumed that x, y, and z axes are arbitrarily set in space. As described above, it is not appropriate to perform movement or rotation on the basis of the arbitrarily set x, y, and z axes. In the disclosed embodiment, axes matching a patient may be set by designating a line segment connecting the right orbitale and the left orbitale as an x'-axis, generating an FH plane using a midpoint between the right porion and the left porion, the right orbitale, and the left orbitale, designating, as a y'-axis, a line segment located on a plane and perpendicular to the x'-axis while the x'-axis is projected onto the FH plane, and designating a line segment perpendicular to the x'-axis and the y'-axis as a z'-axis. The above description of the axes set by reflecting the anatomical features of the patient is only an example, and the present disclosure is not limited thereto.

In various embodiments, the designated x', y' and z' axes may be accurately matched to the x, y and z axes in space, respectively. For example, a 3D image may be accurately positioned in space by rotating and moving the 3D image and landmarks on a screen such that x', y', and z' axes designated based on the landmarks match x, y, and z axes in space.

Hereinafter, an x-axis, a y-axis, and a z-axis to be described below in relation to each operation of a surgery simulation should be understood to mean axes that are set while reflecting anatomical features of a patient. For example, as described above, the x, y and z axes may be understood as either x', y' and z' axes designated based on landmarks of a patient or x, y and z axes accurately matched to the x', y' and z' axes.

The user interface 500 provides a surgery simulation related to a plurality of operations of a surgery on the basis of at least one of an input of numerical values designated by a user or an input of calculated optimal numerical values. In an embodiment, the surgery simulation based on the input of the numerical values designated by the user may be understood to mean that a plurality of operations of a surgery are performed on the basis of the numerical values input by the user through a user interface (i.e., numerical values input by the user in relation to a target surgery level).

The surgery simulation based on the input of the calculated optimal numerical values may be understood to mean that the server 100 analyzes a medical image of a patient or a 3D modeling result of the medical image, i.e., performs anatomical analysis, for automatic calculation of optimal numerical values for corrective surgery, and the plurality of operations are performed on the basis of the calculated numerical values. According to an embodiment, the input of the calculated optimal numerical values may be modified directly by the user to determine final numerical values.

A process of performing a surgery simulation on the basis of at least one of an input of numerical values designated by a user or an input of calculated optimal numerical values will be described in detail below.

In an embodiment, the plurality of operations relate to double-jaw surgery and may include first to sixth operations. The server 100 may provide the surgery simulation by subdividing it into several operations to be applicable to actual surgery. In an embodiment, the first to sixth operations may be performed on the basis of one or more landmarks.

In an embodiment, the plurality of operations may be performed on the basis of data (e.g., a first input to a sixth input) input to a plurality of information input windows included in the user interface 500. In an embodiment, the first to sixth inputs may include a method of rotating an object to be moved (e.g., a part to be corrected by an osteotomy and moved during orthognathic surgery) by inputting the amounts of movement (mm) of one or more landmarks serving as reference points, when not only a movement distance (mm) and a degree of rotation of the object but also a degree of rotational transformation are input.

Hereinafter, a plurality of operations of a surgery provided through a user interface will be described in detail with reference to FIGS. 4 to 9. In an embodiment, a surgery related to the upper jaw will be described as an example in the following drawings and description, but it will be obvious to those of ordinary skill in the art that the surgery is also applicable to various surgical sites, including the lower jaw and the mental region of the jaw, as well as the upper jaw.

Specifically, the plurality of operations may include a first operation S210 of performing movement on an x-axis. The first operation may be related to anterior midline correction.

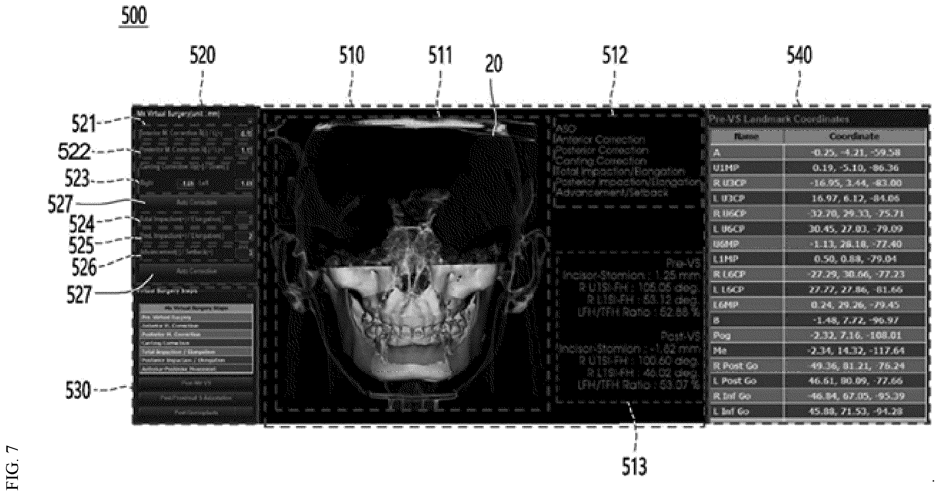

In an embodiment, the user interface 500 may include a first information input window 521 for receiving a first input related to the first operation from a user as shown in FIG. 7. The server 100 may perform the first operation on the basis of the first input that is input from the user through the first information input window 521.

For example, the first input may be information for moving the upper jaw to the left and right when the face is viewed from the front. For example, an input for a negative (-) number on the x-axis may be an input for moving the upper jaw of a patient to the right, and an input for a positive (+) number may be an input for moving the upper jaw of the patient to the left. The above detailed description of the first input is only an example, and the present disclosure is not limited thereto. That is, the server 100 may perform the first operation related to the movement on the x-axis on the basis of the first input that is input to the first information input window 521 of the user interface 500.

The plurality of operations may further include a second operation of performing rotation about a first landmark on the z-axis after applying the first operation based on one or more landmarks. Here, the first landmark may represent the U1MP and may be a midpoint between the right and left maxillary central incisors to be cut.

The second operation (S220) may be related to posterior midline correction. In the second operation, rotation may be yaw-related rotation. In an embodiment, the user interface 500 may include a second information input window 522 for receiving a second input related to the second operation from the user as shown in FIG. 7.

The server 100 may perform the second operation on the basis of the second input that is input from the user through the second information input window 522. For example, the second input may be information for performing rotation about the U1MP on the z-axis until an x-axis coordinate of the U6MP (the center of a line segment connecting a front cusp adjacent to the cheeks to the first molar teeth in the right and left directions) is changed by an input value when the face is viewed from the front.

For example, an input related to a negative (-) number may be an input for performing clockwise rotation about the U1MP on the z-axis, and an input related to a positive (+)number may be an input for performing counterclockwise rotation about the U1MP on the z-axis. The above detailed description of the second input is only an example, and the present disclosure is not limited thereto. That is, the server 100 may perform the second operation related to the rotation about the U1MP on the z-axis on the basis of the second input that is input to the second information input window 522 of the user interface 500.

The plurality of operations may further include a third operation of performing rotation about the second landmark by setting, as an axis of rotation, a line segment generated based on the first and second landmarks, after applying the second operation on the basis of one or more landmarks. Here, the first landmark may be the U1MP, and the second landmark may be the U6MP. That is, the line segment used as the axis of rotation may be a line connecting the U1MP and the U6MP, and in this case, the center of rotation may be the U6MP, which is the second landmark.

The third operation (S230) may be related to canting correction. In an embodiment, the user interface 500 may include a third information input window 523 for receiving a third input related to the third operation from the user as shown in FIG. 7.

The server 100 may perform the third operation on the basis of the third input that is input from the user through the third information input window 523. For example, the third input may be information for performing rotation about the second landmark (i.e., the U6MP) until a z-axis coordinate of the left or right U6CP is changed by an input value, when the face is viewed from the front.

For example, in relation to an input of the amount of movement of the right U6CP, an input related to a negative (-) number may be an input for performing counterclockwise rotation about the U6MP on an axis of rotation, and an input related to a positive (+) number may be an input for performing clockwise rotation about the U6MP on the axis of rotation. The above detailed description of the third input is only an example, and the present disclosure is not limited thereto. That is, the server 100 may perform the third operation related to rotation about the U6MP on the basis of the third input that is input to the third information input window 523 of the user interface 500.

The plurality of operations may further include a fourth operation (S240) of performing movement on the z-axis. The fourth operation may be related to total impaction or elongation. In an embodiment, the user interface 500 may include a fourth information input window 524 for receiving a fourth input related to the fourth operation from the user as shown in FIG. 7.

The server 100 may perform the fourth operation on the basis of the fourth input that is input from the user through the fourth information input window 524. For example, the fourth input may be information for moving the upper jaw upward and downward when the face is viewed from the front.

For example, an input for a negative (-) number on the z-axis may be an input for moving the upper jaw downward (elongation), and an input for a positive (+) number may be an input for moving the upper jaw upward (impaction). The above detailed description of the fourth input is only an example, and the present disclosure is not limited thereto. That is, the server 100 may perform the fourth operation related to the movement on the z-axis on the basis of the fourth input that is input to the fourth information input window 524 of the user interface 500.

The plurality of operations may further include a fifth operation of performing rotation about the first landmark on the x-axis as an axis of rotation, after applying the fourth operation on the basis of one or more landmarks. Here, the first landmark may mean the U1MP.

The fifth operation S250 may be related to posterior impaction or elongation. In the fifth operation, rotation may be pitch-related rotation. In an embodiment, the user interface 500 may include a fifth information input window 525 for receiving a fifth input related to the fifth operation from the user as shown in FIG. 7.

The server 100 may perform the fifth operation on the basis of the fifth input that is input from the user through the fifth information input window 525. For example, the fifth input may be information for performing rotation about the U1MP on the x-axis until a z-axis coordinate of the U6MP is changed by an input value, when the face is viewed from the front.

For example, an input related to a negative (-) number may be an input for performing counterclockwise rotation about the U1MP on the x-axis, and an input related to a positive (+) number may be an input for performing clockwise rotation about the U1MP on the x-axis. The above detailed description of the fifth input is only an example, and the present disclosure is not limited thereto. That is, the server 100 may perform the fifth operation related to rotation about the U1MP on the basis of the fifth input that is input to the fifth information input window 525 of the user interface 500.

The plurality of operations may further include a sixth operation related to movement on the y-axis. The sixth operation (S260) may be related to advancement or setback. In an embodiment, the user interface 500 may include a sixth information input window 526 for receiving a sixth input related to the sixth operation from the user as shown in FIG. 7.

The server 100 may perform the sixth operation on the basis of the sixth input that is input from the user through the sixth information input window 526. For example, the sixth input may be information for moving the upper jaw forward and backward when the face is viewed from the front.

For example, an input for a negative (-) number on the y-axis may be an input for moving the upper jaw forward, and an input for a positive (+) number may be an input for moving the upper jaw backward. The above detailed description of the sixth input is only an example, and the present disclosure is not limited thereto. That is, the server 100 may perform the sixth operation related to the movement on the y-axis on the basis of the sixth input that is input to the sixth information input window 526 of the user interface 500.

As described above, the server 100 may receive numerical information related to the first to sixth operations through a plurality of information input windows included in a numerical value input screen 520 of the user interface 500, and perform the surgery simulation. The surgery simulation may be performed with respect to one or more landmarks related to anatomical points on the head (e.g., the orbital, the ANS, the PNS, the U1MP, the U6MP, etc.), and performed through subdivided operations.

According to an embodiment of the present disclosure, the server 100 may analyze 3D modeling of a patient (i.e., an anatomical analysis) to calculate optimal numerical values for corrective surgery, and perform the plurality of operations on the basis of the calculated numerical values. The server 100 may automatically calculate numerical values for each of the operations on the basis of the coordinates of each of one or more landmarks and perform the plurality of operations on the basis of the calculated numerical values.

That is, the server 100 may calculate optimal numerical values corresponding to the plurality of operations (i.e., the first to sixth operations) through automatic correction, and perform the surgery simulation on the basis of the optimal numerical values. In an embodiment, the calculated numerical values (i.e., set values for movement and rotation) corresponding to the plurality of operations by the server 100 may be values calculated to perform an optimal surgery simulation according to a state of the head of each patient.

In an embodiment, the automatic correction performed by the server 100 may be performed on the basis of a selection input received from a user through an automatic correction input window 527 included in the numerical value input screen 520 of the user interface 500. A method of calculating a plurality of numerical values corresponding to the operations of the surgery by the server 100 to perform an optimal surgery simulation will be described in detail below.

According to an embodiment, the server 100 may calculate an amount of movement on the x-axis required to cause an x-axis coordinate of the U1MP to be the same as those of the nasion (intersection of the frontonasal suture and the internasal suture) so as to obtain a first numerical value corresponding to the first operation. That is, the server 100 may identify a value on the x-axis at which the nasion and the U1MP are the same. Accordingly, when the first numerical value is applied, the U1MP may be located on the same x-axis coordinate as the nasion (i.e., an x-axis coordinate on which a center line on the front teeth is the same as the nasion).

In addition, the server 100 may calculate an amount of movement on the x-axis required to cause an x-axis coordinate of the U6MP to be the same as that of the U1MP while the first numerical value is applied to obtain a second numerical value corresponding to the second operation. In other words, a second numerical value that causes the U1MP and the U6MP to be located on the same x-axis coordinate may be obtained. That is, the second numerical value may be a set value for a midpoint on the front teeth and a midpoint on the rear teeth to be positioned on the same x-axis coordinate. The second numerical value may be a set value for adjustment of rotation related to the torsion, i.e., yaw, of the rear teeth relative to the front teeth. Therefore, a center line of the nasion and a center line of the jaw may be matched to each other through the first and second operations.

In addition, the server 100 may obtain a third numerical value corresponding to the third operation by setting the U6MP as an origin of rotation while the second numerical value is applied, calculating an angle of rotation required to cause a z-axis coordinate of the R U6CP and a z-axis coordinate of the L U6CP to be the same for rotation transformation using the U6MP and the U1MP as the axes of rotation, and calculating the difference between a z-axis coordinate of the U6CP to which the calculated angle of rotation is applied and a z-axis coordinate of the U6CP before the calculated angle of rotation is applied thereto. That is, the third numerical value may be a set value for causing the left teeth and the right teeth to be located at the same z-axis coordinate value when the face is viewed from the front. The third numerical value may be a set value that does not cause a change in the first and second operations but causes the torsion of the left and right teeth to be adjusted through rotation.

Specifically, the U1MP may be located at the right position through the first operation, the U6MP may be located at the right position through the second operation without changing the U1MP, and rotation may be performed about the U1MP and the U6MP as the axes of rotation through the third operation, thereby fixing torsion without causing a change in the U1MP and the U6MP.

In addition, the server 100 may calculate a numerical value for increasing an incisor-stomion value by 2 in a state in which the third numerical value is applied. Here, the incisor-stomion value may be obtained by projecting landmarks such as the stomion (a midpoint on the lips), the R U1CP, and the L U1CP on a midsagittal plane and subtracting a smaller value among the z-axis coordinates of the projected R U1CP and L U1CP from the z-axis coordinate of the projected stomion. That is, the server 100 may obtain a fourth numerical value corresponding to the fourth operation by calculating a numerical value for increasing the incisor-stomion value by 2. That is, the fourth numerical value may be a set value for adjusting a position at which the teeth are shown when the lips are open, i.e., movement on the z-axis.

In addition, the server 100 may calculate a numerical value for causing a U1S1-FH value to be 110 degrees. Here, the U1SI-FH value may be an angle between a line segment connecting the R U1CP and the R U1RP (i.e., a long axis of the right anterior maxilla) and the FH plane. That is, the server 100 may obtain a fifth numerical value corresponding to the fifth operation by rotating the upper jaw about a first origin of rotation on the x-axis so that the U1SI-FH value may be 110 degrees and calculating the difference between a z-axis coordinate of the U6MP to which rotation is applied and a z-axis coordinate of the U6MP before rotation is performed. Here, the first origin of rotation may be an intersection of a line segment connecting the R U1CP and the R U1RP and the FH plane. That is, the fifth numerical value may be a set value for designating a masticatory surface of the upper jaw on the basis of a prominent angle of the front teeth of the upper jaw, i.e., a setting value for adjusting pitch-related rotation.

In addition, the server 100 may obtain a sixth numerical value corresponding to the sixth operation by calculating a numerical value for causing a y-axis coordinate of a point A (a deepest point on a curved surface between the ANS of the upper jaw and the alveolar bone) to be same as a y-axis coordinate of -2 of the nasion in a state in which the fifth numerical value is applied. That is, the sixth numerical value may be a set value for determining an amount of protrusion of the upper jaw.

As described above, the server 100 may calculate a plurality of numerical values corresponding to the operations to perform an optimal surgery simulation. Thus, numerical values related to an ideal virtual surgery can be automatically calculated and provided to improve convenience. In addition, values corresponding to the operations of a surgery are calculated by equations for correcting the upper jaw and the lower jaw on the basis of coordinates of each of one or more landmarks and analysis measurement values, thus guaranteeing reliability.

An existing simulation method is not divided into several operations and provides only results before and after an object to be moved is moved, thus making it difficult to systematically plan a surgery, reproduce a surgery simulation or explain a goal of the surgery intended by a user. To address the above-described problems, surgery simulation methods according to embodiments set forth herein provide a convenient and systematic surgery simulation function and allow a user to easily explain a goal of a surgery applied in each operation of the surgery.

According to an embodiment of the present disclosure, the server 100 may generate surgery simulation result data by performing a surgery simulation on the basis of a response to the user interface, received from the user terminal (S130).

In an embodiment, the surgery simulation result data may include postoperative 3D modeling data, progress data, and main diagnostic index data. Specifically, the surgery simulation result data may include postoperative 3D modeling data obtained as a result of performing the surgery simulation.

In an embodiment, the server 100 may display 3D modeling data 20 on a data display screen 510 as shown in FIG. 7. Specifically, the 3D modeling data 20 may be displayed on a 3D modeling data display screen 511 of the data display screen 510.

The 3D modeling data display screen 511 may display the 3D modeling data 20 that changes as each of the plurality of operations is performed. For example, when the last operation is completed, the 3D modeling data 20, which is postoperative 3D modeling data, may be displayed on the 3D modeling data display screen 511.

The surgery simulation result data may further include progress data indicating which operation among the plurality of operations is being currently performed. In an embodiment, the progress data may be provided while being displayed on a surgical operation display screen 530. The surgical operation display screen 530 may display the plurality of operations and allow the 3D modeling data display screen 511 to be changed on the basis of a selection input received from a user for at least one of the plurality of operations. For example, 3D modeling data obtained after the fifth operation is performed may be output to the 3D modeling data display screen 511 on the basis of a selection input received from a user related to posterior impaction or elongation (the fifth operation).

That is, the 3D modeling data display screen 511 may display an operation of a surgery performed on the basis of user inputs related to the numerical value input screen 520 and the surgical operation display screen 530 through a simulation based on the 3D modeling data.

In an embodiment, the data display screen 510 may include a first information display screen 512 and a second information display screen 513. The first information display screen 512 may include information for checking operations of a surgery applied to current 3D modeling data output to the 3D modeling data display screen 511.

For example, as shown in FIG. 7, when all of anterior correction, posterior correction, canting correction, total impaction or elongation, posterior impaction or elongation, and advancement or setback are displayed on the first information display screen 512, the current 3D modeling data displayed on the 3D modeling data display screen 511 may be 3D modeling data (i.e., postoperative 3D modeling data) to which the first to sixth operations, i.e., all of the plurality of operations, are applied.

The second information display screen 513 may be an area that displays 3D modeling data currently displayed on the 3D modeling data display screen 511 and a numerical analysis result before virtual surgery is applied. That is, the first information display screen 512 and the second information display screen 513 may display an operation that is being performed among operations of a process of a surgery during a virtual surgery simulation, and show a comparison between analysis results before and after each of the operations of the surgery is performed, thereby facilitating a process of planning and checking a surgery.

Specifically, an analysis of numerical values, such as the incisor-stomion (obtained by measuring the difference in height between an end of the front teeth of the upper jaw and a midpoint on the lips, and an exposed area of the front teeth of the upper jaw when the lips are open), the R U1SI-FH (obtained by measuring an angle between the right front teeth of the upper jaw and the FH plane, and a prominent angle of the front teeth of the upper plane), the R L1SI-FH (obtained by measuring an angle between the right front teeth of the lower jaw and the FH plane, and a prominent angle of the front teeth of the lower jaw), the LFH/TFH (obtained by measuring a ratio of the position of the jaw to the length of the face, and the position of the jaw in the face), before and after virtual surgery is applied may be visualized on a screen to provide indexes required to plan a surgery.

The surgery simulation result data may include main diagnostic index data related to an amount of change in one or more landmarks when the surgery simulation is performed.

As illustrated in FIG. 7, the server 100 may display the main diagnosis index data on a diagnostic index data display screen 540. The main diagnostic index data display screen 540 may display numerical information, including a state of each landmark before movement (a preoperative state), a state of each landmark after movement (a state in which an operation currently displayed on a screen is applied), the difference between positions of each landmark before and after movement, etc., up to two decimal places on a screen.

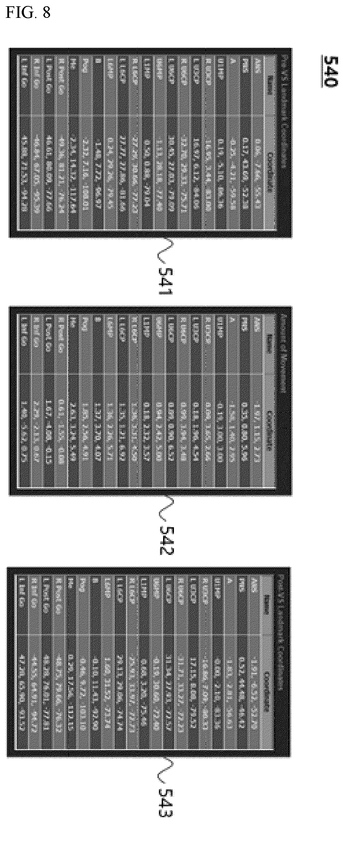

In an embodiment, information displayed on the diagnostic index data display screen 540 may include first diagnostic index data 541, second diagnostic index data 542, and third diagnostic index data 543 as shown in FIG. 8. In an embodiment, the first diagnostic index data 541 may include information regarding a position of each of one or more landmarks that are in the original state before the operations are applied.

The second diagnostic indicator data 542 may include information regarding an amount of change in the one or more landmarks after the operations are applied.

The third diagnostic index data 543 may include information regarding the position of each of the one or more landmarks changed by an operation currently applied to a screen. In other words, the server 100 may display various types of diagnostic index data, i.e., information about a position of each landmark before/after each of the operations is applied and a total amount of change of each landmark, on the diagnostic index data display screen 540.

In various embodiments, a user (i.e., a person who will perform surgery) may be provided with information to be applied to actual surgery through information displayed on the diagnostic index data display screen 540. For example, the user may obtain information about an amount of change in a second landmark on the basis of a first landmark and the like to perform a specific operation.

Accordingly, the server 100 may perform rotation and movement using axes of rotation according to an anatomical positional relationship of a patient on the basis of clear criteria (anatomical points) rather than performing a surgery simulation by movement, rotation, etc. during a surgical process using 3D modeling data (e.g., using x, y and z axes that are vague criteria designated on a screen by a user with a mouse or the like during the simulation) without clear criteria.

That is, the server 100 may provide a virtual surgery simulation using axes and an origin of rotation that are differently set for each patient on the basis of an actual anatomical position and shape of each patient rather than virtual axes and a virtual origin of rotation. Therefore, an optimal surgery simulation can be performed according to a current state of each patient.

Additionally, the server 100 may perform a surgery simulation on the basis of a plurality of operations of a surgery, and generate various types of simulation result data related to the progress of the surgery for each of the plurality of operations.

Therefore, a patient-customized surgery simulation can be performed while reflecting current anatomical features of each patient, and rotation and movement can be performed for each of operations of a surgery during the simulation so that the goal of each rotation and movement intended by a person who will perform surgery can be checked to systematically plan and analyze the surgery.

According to an embodiment of the present disclosure, the server 100 may generate splint modeling data on the basis of 3D modeling data. A splint is used in orthognathic surgery, and may be a teeth-shaped frame, e.g., a mouthpiece, which is manufactured in a horseshoe shape by filling a space between the upper teeth and the lower teeth with a composite material (e.g., resin). The splint may be fixed on teeth of the uncised jaw during surgery to set teeth of the incised jawbone to a desired position. For example, the splint may include an intermediate splint for setting a position of the upper jaw and a final splint for setting a position of the lower jaw.

In an embodiment, in actual surgery, an object to be moved is moved to and fixed on a target position in various ways according to a surgical method employed by a person who will perform the surgery rather than moving the object through six operations of a virtual surgery simulation. In this case, a position of the object can be easily set using a surgical splint.

That is, a virtual surgery simulation can be performed using axes and an origin of rotation based on anatomical points of a patient to plan a surgery more medically and clearly and perform a simulation in a 3D environment more accurately and reproducibly. Thereafter, in actual surgery, an object to be moved can be moved to a target position determined through the surgery simulation, but a method thereof is not limited and the splint described above can be moved.

Accordingly, the server 100 may generate splint modeling data 30 for manufacturing the splint on the basis of modeling data (i.e., postoperative 3D modeling data) obtained after performing the virtual surgery simulation including six operations of a surgery.

Specifically, the server 100 may identify postoperative 3D modeling data 20 included in surgery simulation result data. The server 100 may generate the splint modeling data 30 on the basis of the postoperative 3D modeling data 20.

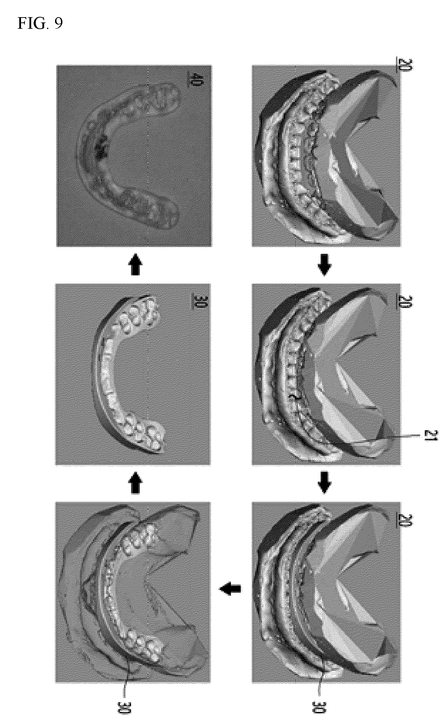

Referring to FIG. 9, the position of an upper jaw part may be changed according to a result of a surgery simulation including a plurality of operations of a surgery as described above. In this case, a space 21 between the upper teeth and the lower teeth may be filled with a virtual composite material to perform modeling of a splint according to a shape of the teeth. In an embodiment, the generated splint modeling data 30 may be a basis for generation of an actual splint 40.

The actual splint 40 to be used for an actual surgery may be manufactured through the splint modeling data 30. In other words, the server 100 may perform a surgery simulation including six operations of a surgery and generate the splint modeling data 30 required to manufacture the actual splint 40 on the basis of information obtained as a result of performing the surgery simulation. Accordingly, convenience can be increased during the manufacture of a splint, a splint can be manufactured very precisely without depending on manual work, and furthermore, the stability and accuracy of surgery can be improved by applying the splint to actual surgery.

According to various embodiments of the present disclosure, an orthognathic simulation can be performed to systematically plan operations of a surgery on the basis of singularities (landmarks such as the anterior nasal spine (ANS)) of an anatomical structure of each patient so that the goal of the surgery can be explained between a doctor and a patient or between doctors and the same result can be reproduced using numerical values for each of the operations.

Effects of the present disclosure are not limited to the above effect, and other effects that are not described above will be clearly understood by those of ordinary skill in the art from the above detailed description.

The operations of the method or an algorithm described above in connection with embodiments of the present disclosure may be implemented directly by hardware, a software module executed by hardware, or a combination thereof. The software module may reside in a RAM, a ROM, an EPROM, an EEPROM, a flash memory, a hard disk, a removable disk, a CD-ROM, or a type of computer-readable recording medium well-known in the technical field to which the present disclosure pertains.

Components of the present disclosure may be embodied in the form of a program (or an application) and stored in a medium to be executed in combination with a computer which is hardware. The components of the present disclosure may be implemented by software programming or software elements, and similarly, embodiments may be implemented in a programming or scripting language such as C, C++, Java, or an assembler, including data structures, processes, routines, or various algorithms which are combinations of other programming components. Functional aspects may be embodied as an algorithm executable by one or more processors.

It will be understood by those of ordinary skill in the art that various types of logic blocks, modules, processors, means, circuits, and operations of algorithms described above as examples in relation to the embodiments set forth herein are implementable using electron hardware, various types of programs or design code (referred to as "software" herein for convenience of description), or a combination thereof. To clearly describe the interoperability between hardware and software, various types of components, blocks, modules, circuits, and operations have been generally described above as examples in relation to functions thereof. Whether such a function is implemented as hardware or software depends on a specific application and design restrictions imposed on the entire system. Functions described above for each specific application can be implemented in various ways by those of ordinary skill in the art but decisions of the implementation should not be understood as not falling within the scope of the present disclosure.

The various embodiments set forth herein may be implemented as articles manufactured by methods, apparatuses, or standard programming and/or engineering techniques. The term "manufactured article" should be understood to include a computer program, a carrier or media accessible by any computer-readable device. Examples of a computer-readable medium may include, but are not limited to, magnetic storage devices (e.g., a hard disk, a floppy disk, a magnetic strip, etc.), optical disks (e.g., a CD, a DVD, etc.), smart cards, and flash memory devices (e.g., an EEPROM, a card, a stick, a key drive, etc.). In addition, the various types of storage media presented herein include one or more devices for storing information and/or other machine-readable media. The term "machine-readable media" includes, but is not limited to, wireless channels and various other media for storing, retaining, and/or transmitting instruction(s) and/or data.

It should be understood that the specific order or hierarchy of operations of each of the presented processes is an example of exemplary approaches. It should be understood that a specific order of hierarchical structure of operations of a process within the scope of the present disclosure may be rearranged on the basis of design priorities. The appended method claims provide elements of various operations in a sample order but should not be understood as being limited to the specific order or hierarchical structure presented herein.

A description of embodiments set forth herein is provided to help those of ordinary skill in the art use or implement the present disclosure. It will be obvious to those of ordinary skill in the technical field of the present disclosure that various modifications may be made in these embodiments, and the general principles defined herein may be applied to other embodiments without departing from the scope of the present invention as defined by the claims.

## Claims

1. A method of providing a surgery simulation, which is performed by one or more processors of a computing device, the method comprising:
generating three-dimensional (3D) modeling data on the basis of a medical image (S110);
generating a user interface including the 3D modeling data, and providing the user interface to a user terminal (S120); and
generating surgery simulation result data by performing the surgery simulation on the basis of a response to the user interface, the response being received from the user terminal (S130);
wherein the generating of the 3D modeling data comprises:
identifying one or more landmarks on the medical image; and
generating the 3D modeling data by performing segmentation on the medical image; and
wherein the generating of the surgery simulation result data comprises:
setting axes and reference points corresponding to the 3D modeling data on the basis of the one or more landmarks;
determining an object to be moved for surgery; and
performing the surgery simulation by moving and rotating the object on the basis of the set axes and reference points, wherein the surgery simulation comprises a plurality of operations of a surgery, the plurality of operations of the surgery comprising:
a first operation (S210) of moving the object on a first axis;
a second operation (S220) of rotating the object about a first landmark on a second axis;
a third operation (S230) of rotating the object about a second landmark on a third axis, the third axis being a line segment generated on the basis of the first landmark and the second landmark;
a fourth operation (S240) of moving the object on the second axis;
a fifth operation (S250) of rotating the object by setting the first landmark as an origin of rotation and the first axis as an axis of rotation; and
a sixth operation (S260) of moving the object on a fourth axis.

2. The method of claim 1, wherein the axes and the reference points corresponding to the 3D modeling data comprise at least one of a point, a line, or a plane generated after performing an operation related to at least one of a midpoint, an intersection, a line segment, a plane, a vertical relationship, or a parallel relationship based on the one or more landmarks.

3. The method of claim 1 or 2, wherein the generating of the surgery simulation result data comprises performing the surgery simulation including the plurality of operations of the surgery on the basis of at least one of an input of a numerical value designated by a user or an input of a calculated optimal numerical value.

4. The method of any one of the preceding claims, wherein the user interface comprises
one or more input windows for receiving a first input to a sixth input from a user for the first to sixth operations (S210, S220, S230, S240, S250, S260), and
the plurality of operations of the surgery comprise performing a simulation including the first to sixth operations (S210, S220, S230, S240, S250, S260) on the basis of the first to sixth inputs from the user.

5. The method of any one of the preceding claims, wherein the surgery simulation result data comprises main diagnostic index data related to at least one of postoperative 3D modeling data obtained as a result of performing the surgery simulation, progress data indicating which operation among the plurality of operations of the surgery is being performed, positions and amounts of change of the one or more landmarks as the surgery simulation is performed, or a numerical analysis of a change in physiognomy using the changed one or more landmarks.

6. The method of any one of the preceding claims, further comprising generating splint modeling data on the basis of the 3D modeling data,
wherein the generating of the splint modeling data comprises:
identifying postoperative 3D modeling data included in the surgery simulation result data; and
generating the splint modeling data on the basis of the postoperative 3D modeling data.

7. An apparatus (100) comprising:
a memory (120) storing one or more instructions; and
a processor (110) configured to execute the one or more instructions stored in the memory (120) to perform the method of any one of the preceding claims.

8. A computer program (151) which when executed by a processor (110) is arranged to perform the method of any one of claims 1 to 6.

## Patentansprüche

1. Verfahren zum Bereitstellen einer chirurgischen Simulation, die durch einen oder mehrere Prozessoren einer Rechenvorrichtung durchgeführt wird, wobei das Verfahren Folgendes umfasst:
Erzeugen von dreidimensionalen (3D) Modellierungsdaten auf der Basis eines medizinischen Bildes (S110);
Erzeugen einer Benutzerschnittstelle, welche die 3D-Modellierungsdaten beinhaltet, und Bereitstellen der Benutzerschnittstelle an ein Benutzerendgerät (S120); und
Erzeugen von chirurgischen Simulationsergebnisdaten durch Durchführen der chirurgischen Simulation auf der Basis einer Reaktion auf die Benutzerschnittstelle, wobei die Reaktion von dem Benutzerendgerät (S130) empfangen wird;
wobei das Erzeugen der 3D-Modellierungsdaten Folgendes umfasst:
Identifizieren von einem oder mehreren Orientierungspunkten auf dem medizinischen Bild; und
Erzeugen der 3D-Modellierungsdaten durch Durchführen von Segmentierung an dem medizinischen Bild; und
wobei das Erzeugen der chirurgischen Simulationsergebnisdaten Folgendes umfasst:
Festlegen von Achsen und Referenzpunkten entsprechend den 3D-Modellierungsdaten auf der Basis von dem einen oder den mehreren Orientierungspunkten;
Bestimmen eines zu bewegenden Objekts für Chirurgie; und
Durchführen der chirurgischen Simulation durch Bewegen und Drehen des Objekts auf der Basis der festgelegten Achsen und Referenzpunkte, wobei die chirurgische Simulation eine Vielzahl von Vorgängen einer Chirurgie umfasst, wobei die Vielzahl von Vorgängen der Chirurgie Folgendes umfasst:
einen ersten Vorgang (S210) des Bewegens des Objekts auf einer ersten Achse;
einen zweiten Vorgang (S220) des Drehens des Objekts um einen ersten Orientierungspunkt auf einer zweiten Achse;
einen dritten Vorgang (S230) des Drehens des Objekts um einen zweiten Orientierungspunkt auf einer dritten Achse, wobei die dritte Achse ein Liniensegment ist, das auf der Basis des ersten Orientierungspunktes und des zweiten Orientierungspunktes erzeugt wird;
einen vierten Vorgang (S240) des Bewegens des Objekts auf der zweiten Achse;
einen fünften Vorgang (S250) des Drehens des Objekts durch Festlegen des ersten Orientierungspunktes als Ursprung von Drehung und der ersten Achse als Drehachse; und
einen sechsten Vorgang (S260) des Bewegens des Objekts auf einer vierten Achse.

2. Verfahren nach Anspruch 1, wobei die Achsen und die Referenzpunkte, die den 3D-Modellierungsdaten entsprechen, zumindest eines von einem Punkt, einer Linie oder einer Ebene umfassen, der/die nach Durchführen eines Vorgangs in Bezug auf zumindest eines von einem Mittelpunkt, einem Schnittpunkt, einem Liniensegment, einer Ebene, einer vertikalen Beziehung oder einer parallelen Beziehung basierend auf dem einen oder den mehreren Orientierungspunkten erzeugt wird.

3. Verfahren nach Anspruch 1 oder 2, wobei das Erzeugen der chirurgischen Simulationsergebnisdaten Durchführen der chirurgischen Simulation, welche die Vielzahl von Vorgängen der Chirurgie beinhaltet, auf der Basis von zumindest einem von einer Eingabe eines numerischen Wertes, der durch einen Benutzer benannt ist, oder einer Eingabe eines berechneten optimalen numerischen Wertes umfasst.

4. Verfahren nach einem der vorhergehenden Ansprüche, wobei die Benutzerschnittstelle Folgendes umfasst:
ein oder mehrere Eingabefenster zum Empfangen einer ersten Eingabe bis einer sechsten Eingabe von einem Benutzer für den ersten bis sechsten Vorgang (S210, S220, S230, S240, S250, S260), und
die Vielzahl von Vorgängen der Chirurgie Durchführen einer Simulation, welche den ersten bis sechsten Vorgang (S210, S220, S230, S240, S250, S260) beinhaltet, auf der Basis der ersten bis sechsten Eingabe von dem Benutzer umfasst.

5. Verfahren nach einem der vorhergehenden Ansprüche, wobei die chirurgischen Simulationsergebnisdaten Hauptdiagnoseindexdaten umfassen, die sich auf zumindest eines von postoperativen 3D-Modellierungsdaten, die als Ergebnis des Durchführens der chirurgischen Simulation erhalten werden, Fortschrittsdaten, die angeben, welcher Vorgang aus der Vielzahl von Vorgängen der Chirurgie durchgeführt wird, Positionen und Mengen an Änderung des einen oder der mehreren Orientierungspunkte, während die chirurgische Simulation durchgeführt wird, oder einer numerischen Analyse einer Änderung der Physiognomie unter Verwendung des einen oder der mehreren geänderten Orientierungspunkte beziehen.

6. Verfahren nach einem der vorhergehenden Ansprüche, ferner umfassend Erzeugen von Schienenmodellierungsdaten auf der Basis der 3D-Modellierungsdaten,
wobei das Erzeugen der Schienenmodellierungsdaten Folgendes umfasst:
Identifizieren von postoperativen 3D-Modellierungsdaten, die in den chirurgischen Simulationsergebnisdaten enthalten sind; und
Erzeugen der Schienenmodellierungsdaten auf der Basis der postoperativen 3D-Modellierungsdaten.

7. Einrichtung (100), umfassend:
einen Speicher (120), der eine oder mehrere Anweisungen speichert; und
einen Prozessor (110), der konfiguriert ist, um die eine oder mehreren Anweisungen auszuführen, die in dem Speicher (120) gespeichert sind, um das Verfahren nach einem der vorhergehenden Ansprüche durchzuführen.

8. Computerprogramm (151), das, wenn durch einen Prozessor (110) ausgeführt, angeordnet ist, um das Verfahren nach einem der Ansprüche 1 bis 6 durchzuführen.

## Revendications

1. Procédé de fourniture d'une simulation chirurgicale, qui est réalisée par un ou plusieurs processeurs d'un dispositif informatique, le procédé comprenant :
la génération de données de modélisation tridimensionnelle (3D) sur la base d'une image médicale (S110) ;
la génération d'une interface utilisateur comprenant les données de modélisation 3D, et la fourniture de l'interface utilisateur à un terminal utilisateur (S120) ; et
la génération de données de résultat de simulation chirurgicale en réalisant la simulation chirurgicale sur la base d'une réponse à l'interface utilisateur, la réponse étant reçue en provenance du terminal utilisateur (S130) ;
ladite génération des données de modélisation 3D comprenant :
l'identification d'un ou plusieurs repères sur l'image médicale ; et
la génération des données de modélisation 3D en réalisant une segmentation sur l'image médicale ; et
ladite génération des données de résultat de simulation chirurgicale comprenant :
la définition d'axes et de points de référence correspondant aux données de modélisation 3D sur la base du ou des repères ;
la détermination d'un objet à déplacer pour la chirurgie ; et
la réalisation de la simulation chirurgicale en déplaçant et en faisant tourner l'objet sur la base des axes et des points de référence définis, ladite simulation chirurgicale comprenant une pluralité d'opérations d'une chirurgie, la pluralité d'opérations de la chirurgie comprenant :
une première opération (S210) de déplacement de l'objet sur un premier axe ;
une deuxième opération (S220) de rotation de l'objet autour d'un premier repère sur un deuxième axe ;
une troisième opération (S230) de rotation de l'objet autour d'un second repère sur un troisième axe, le troisième axe étant un segment de ligne généré sur la base du premier repère et du second repère ;
une quatrième opération (S240) de déplacement de l'objet sur le deuxième axe ;
une cinquième opération (S250) de rotation de l'objet en définissant le premier repère en tant qu'origine de rotation et le premier axe en tant qu'axe de rotation ; et
une sixième opération (S260) de déplacement de l'objet sur un quatrième axe.

2. Procédé de la revendication 1, lesdites axes et lesdits points de référence correspondant aux données de modélisation 3D comprenant au moins l'un d'un point, d'une ligne ou d'un plan généré après avoir réalisé une opération liée à au moins l'un d'un milieu, d'une intersection, d'un segment de ligne, d'un plan, d'une relation verticale ou d'une relation parallèle sur la base du ou des repères.

3. Procédé de la revendication 1 ou 2, ladite génération des données de résultat de simulation chirurgicale comprenant la réalisation de la simulation chirurgicale comprenant la pluralité d'opérations de la chirurgie sur la base d'au moins l'une d'une entrée d'une valeur numérique désignée par un utilisateur ou d'une entrée d'une valeur numérique optimale calculée.

4. Procédé de l'une quelconque des revendications précédentes, ladite interface utilisateur comprenant
une ou plusieurs fenêtres d'entrée destinées à recevoir une première entrée à une sixième entrée en provenance d'un utilisateur pour les première à sixième opérations (S210, S220, S230, S240, S250, S260), et
ladite pluralité d'opérations de la chirurgie comprenant la réalisation d'une simulation comprenant les première à sixième opérations (S210, S220, S230, S240, S250, S260) sur la base des première à sixième entrées de l'utilisateur.

5. Procédé de l'une quelconque des revendications précédentes, lesdites données de résultat de simulation chirurgicale comprenant des données d'indice de diagnostic principales liées à au moins l'une des données de modélisation 3D postopératoires obtenues à la suite de la réalisation de la simulation chirurgicale, des données d'avancement indiquant quelle opération parmi la pluralité d'opérations de la chirurgie est réalisée, des positions et des quantités de changement du ou des repères au fur et à mesure que la simulation chirurgicale est réalisée, ou d'une analyse numérique d'un changement de physionomie à l'aide du ou des repères modifiés.

6. Procédé de l'une quelconque des revendications précédentes, comprenant en outre la génération de données de modélisation d'attelle sur la base des données de modélisation 3D,
ladite génération des données de modélisation d'attelle comprenant :
l'identification de données de modélisation 3D postopératoires comprises dans les données de résultat de simulation chirurgicale ; et
la génération des données de modélisation d'attelle sur la base des données de modélisation 3D postopératoires.

7. Appareil (100), comprenant :
une mémoire (120) stockant une ou plusieurs instructions ; et
un processeur (110) configuré pour exécuter la ou les instructions stockées dans la mémoire (120) pour réaliser le procédé de l'une quelconque des revendications précédentes.

8. Programme informatique (151) qui, lorsqu'il est exécuté par un processeur (110), est agencé pour réaliser le procédé de l'une quelconque des revendications 1 à 6.
